# EUROPÄISCHE PATENTANMELDUNG

(11) **EP 2 008 708 A2**
(43) Veröffentlichungstag der Anmeldung: **31.12.2008**
(21) Anmeldenummer: 08011314.5
(22) Anmeldetag: 21.06.2008
(51) Int. Cl.: B01F 17/42, A61K 8/86, A61K 9/08

(54) **Flüssige Lösungsvermittler enthaltend ethoxylierte Fettalkohole und ethoxylierte Triglyceride**

(30) Priorität: 29.06.2007 DE 102007030434
(71) Anmelder: Clariant International Ltd., 4132 Muttenz (CH)
(72) Erfinder: Johannpeter, Wiebke, 61449 Steinbach am Taunus (DE); Stelter, Wibke, Dr., 61184 Großkarben (DE)
(74) Vertreter: Paczkowski, Marcus

(57) **Zusammenfassung**

Es werden Lösungsvermittler beschrieben, die
a) ein oder mehrere ethoxylierte Fettalkohole gemäß der Formel (I) 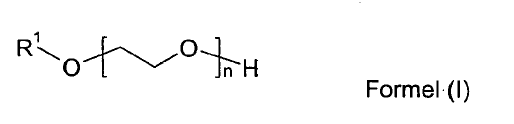 worin R¹ für einen linearen oder verzweigten Alkylrest mit 6 bis 12 Kohlenstoffatomen und n im Durchschnitt für eine Zahl von 2 bis 12 steht,
b) ein oder mehrere ethoxylierte Triglyceride deren Acylreste sich von linearen oder verzweigten gesättigten Fettsäuren mit 6 bis 22 Kohlenstoffatomen ableiten, und
c) Wasser
enthalten und dadurch gekennzeichnet sind, dass sie bei Raumtemperatur flüssig und klar sind und keine Anlagerungsprodukte von Ethylenoxid und gleichzeitig Propylenoxid an Fettalkohole enthalten.

Die Lösungsvermittler eignen sich insbesondere zur Herstellung von kosmetischen, dermatologischen oder pharmazeutischen Formulierungen.

## Beschreibung

Die vorliegende Erfindung betrifft neue Lösungsvermittler mit klarem Aussehen und sehr gutem Lösungsvermögen von lipophilen Stoffen in Zusammensetzungen mit hohem Wassergehalt und deren Verwendung in kosmetischen, dermatologischen und pharmazeutischen Formulierungen.

Lipophile Stoffe, wie beispielsweise Vitamine, Parfümöle oder UV-Lichtschutzfilter, lassen sich vielfach nur schwer in kosmetische oder pharmazeutische Zubereitungen einarbeiten, insbesondere dann, wenn diese einen hohen Wassergehalt aufweisen. In solchen Fällen kommen Lösungsvermittler, auch Hydrotrope genannt, zum Einsatz. Bei diesen handelt es sich um einzelne Stoffe oder Mischungen mit mittleren HLB-Werten, die in der Lage sind, eine Brücke von der polaren Umgebung zum unpolaren Substrat zu bilden. Sehr effektive Hydrotrope stellen die Sulfonate kurzkettiger Alkylaromaten, wie z. B. Toluol- oder Cumolsulfonat, dar, die wegen ihrer unzureichenden, hautkosmetischen Verträglichkeit aber im Bereich der Kosmetik keine Bedeutung haben. Andere kosmetische Solubilisatoren, wie z. B. spezielle, hydrophilisierte Öle, sind zwar hautverträglich, besitzen aber kein ausreichendes Lösungsvermögen und/oder ein schlechtes Kälteverhalten, d. h. sie zeigen schon bei Raumtemperatur die Tendenz zur Austrübung.

In US 2004/0043045 werden kosmetische Zubereitungen, bestehend aus 40 bis 99 Gew.-% eines Fettalkohols mit 6 bis 22 Kohlenstoffatomen und 1 bis 60 Gew.-% an lipophilen Komponenten, beispielsweise ethoxylierte Partialglyceride, und die Verwendung von Fettalkoholen als Lösungsvermittler für Aktivsubstanzen mit geringer Wasserlöslichkeit, beschrieben. Fettalkohole sind jedoch als Lösungsvermittler zur Herstellung von klaren und flüssigen Produkten ungeeignet.

JP 09301844 offenbart, dass Mischungen aus ethoxyliertem Octyldodecylalkohol mit 10 bis 25 Ethylenoxideinheiten und ethoxyliertem und hydriertem Rizinusöl mit 40 bis 80 Ethylenoxid-Einheiten als Lösungsvermittler für lipophile Komponenten in wässrigen Systemen geeignet sind. Auf Grund des hohen Ethoxylierungsgrades von 10 bis 25 und des langen Fettalkoholrestes von 20 Kohlenstoffatomen im ethoxylierten Octyldodecylalkohol und des damit verbundenen relativ hohen Molekulargewichtes zeigen diese Mischungen aber auch eine relativ hohe Viskosität und dementsprechend eine verbesserungsbedürftige Handhabung.

In DE 100 25 756 werden Mischungen aus a) ethoxylierten Fettalkoholen, bevorzugt ethoxylierten C₁₂-C₁₈-Fettalkoholen, b) ethoxylierten/propoxylierten Fettalkoholen und c) ethoxylierten Triglyceriden als Lösungsvermittler offenbart. Diese Mischungen sind flüssig, zeigen ein gutes Lösungsvermögen und zeichnen sich durch ein klares Aussehen aus. Propoxylierte Fettalkohole sind allerdings biologisch nicht gut abbaubar.

Die Aufgabe der vorliegenden Erfindung hat somit darin bestanden, neue Lösungsvermittler zur Verfügung zu stellen, die ein sehr gutes Lösungsvermögen, insbesondere gegenüber lipophilen Stoffen, wie z. B. Parfümölen, Vitaminen, UV-Lichtschutzfiltern und dergleichen, aufweisen, bei Raumtemperatur flüssig und klar sind, einen Kältetrübungspunkt unterhalb von 10 °C aufweisen, toxikologisch und ökotoxikologisch unbedenklich sind und ein gutes Hautgefühl vermitteln.

Überraschenderweise wurde gefunden, dass Mischungen von ethoxylierten Fettalkoholen, gekennzeichnet durch kürzerkettige Fettalkoholreste mit 6 bis 12 Kohlenstoffatomen und einen durchschnittlichen Ethoxylierungsgrad von 12 oder weniger mit ethoxylierten Triglyceriden und Wasser ein sehr gutes Lösungsvermögen, insbesondere gegenüber Parfümölen, Vitaminen und UV-Lichtschutzfiltern, aufweisen und gleichzeitig in ökotoxikologischer Sicht vorteilhafter sind als Produkte des Standes der Technik. Ein weiterer Vorteil besteht darin, dass die Mischungen bei Raumtemperatur flüssig und klar sind, sich daher leicht verarbeiten lassen, den unter ihrer Verwendung hergestellten kosmetischen Produkten ein ästhetisches Aussehen verleihen und zudem einen Kältetrübungspunkt im gewünschten Bereich, nämlich unter 10 °C, aufweisen.

Gegenstand der Erfindung sind Lösungsvermittler, enthaltend
a) ein oder mehrere ethoxylierte Fettalkohole gemäß der Formel (I) 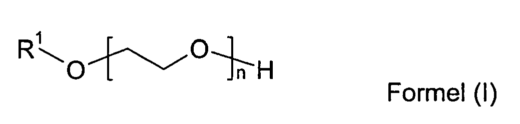 worin R¹ für einen linearen oder verzweigten Alkylrest mit 6 bis 12, vorzugsweise 8 bis 12, und besonders bevorzugt 11 Kohlenstoffatomen und n im Durchschnitt für eine Zahl von 2 bis 12, vorzugsweise 5 bis 11, besonders bevorzugt 6 bis 9, und insbesondere bevorzugt 8, steht,
b) ein oder mehrere ethoxylierte Triglyceride deren Acylreste sich von linearen oder verzweigten gesättigten Fettsäuren mit 6 bis 22 und vorzugsweise 12 bis 18 Kohlenstoffatomen ableiten, und
c) Wasser,
dadurch gekennzeichnet, dass der Lösungsvermittler bei Raumtemperatur flüssig und klar ist und keine Anlagerungsprodukte von Ethylenoxid und gleichzeitig Propylenoxid an Fettalkohole enthält.

In einer bevorzugten Ausführungsform der Erfindung enthalten die Lösungsvermittler mehrere ethoxylierte Fettalkohole gemäß der Formel (I), insbesondere ein Gemisch aus linearen und verzweigten ethoxylierten Fettalkoholen, wobei das Gewichtsverhältnis von ethoxylierten Fettalkoholen der Formel (I) mit linearen Resten R¹ zu ethoxylierten Fettalkoholen der Formel (I) mit verzweigten Resten R¹ von 40: 60 bis 60 : 40, vorzugsweise von 45 : 55 bis 55 : 45, und besonders bevorzugt 50: 50, beträgt.

In einer weiteren bevorzugten Ausführungsform der Erfindung enthalten die Lösungsvermittler einen oder mehrere ethoxylierte Fettalkohole gemäß der Formel (1), worin R¹ ein linearer oder verzweigter Alkylrest mit 11 Kohlenstoffatomen ist.

In einer weiteren bevorzugten Ausführungsform der Erfindung enthalten die Lösungsvermittler einen oder mehrere ethoxylierte Fettalkohole gemäß der Formel (I), worin n im Durchschnitt für eine Zahl von 6 bis 9 und vorzugsweise 8 steht.

In einer weiteren bevorzugten Ausführungsform der Erfindung enthalten die Lösungsvermittler ein oder mehrere ethoxylierte Triglyceride der Komponente b) ausgewählt aus Anlagerungsprodukten von durchschnittlich 20 bis 100 Mol, vorzugsweise 30 bis 80 Mol, besonders bevorzugt 35 bis 65 Mol und insbesondere bevorzugt 40 Mol Ethylenoxid an Triglyceride, insbesondere an hydriertes Castoröl.

Die erfindungsgemäßen Lösungsvermittler sind flüssig und klar und zeichnen sich durch Kälte-Trübungspunkte unterhalb von +10 °C aus.

In einer weiteren bevorzugten Ausführungsform der Erfindung enthalten die Lösungsvermittler die ethoxylierten Fettalkohole der Komponente a) in einer Menge von 20 bis 70 Gew.-%, vorzugsweise von 30 bis 65 Gew.-% und besonders bevorzugt von 33 bis 60 Gew.-%, die ethoxylierten Triglyceride der Komponente b) in einer Menge von 20 bis 60 Gew.-% , vorzugsweise von 25 bis 55 Gew.-% und besonders bevorzugt von 26 bis 51 Gew.-% und Wasser in einer Menge von 1 bis 20 Gew.-%, vorzugsweise von 5 bis 18 Gew.-% und besonders bevorzugt von 9 bis 16 Gew.-%.

In einer besonders bevorzugten Ausführungsform der Erfindung sind die Lösungsvermittler frei von Propylenoxid-haltigen Komponenten.

In einer insbesondere bevorzugten Ausführungsform der Erfindung bestehen die Lösungsvermittler aus den ethoxylierten Fettalkoholen der Komponente a), den ethoxylierten Triglyceriden der Komponente b) und Wasser.

In einer weiteren bevorzugten Ausführungsform der Erfindung zeichnen sich die flüssigen Lösungsvermittler aus durch Viskositäten bei 22,5 °C (RV Brookfield Viskosimeter, 20 U/min, Spindel 3), die im Bereich von 100 bis 700 mPa·s, bevorzugt im Bereich von 100 bis 600 mPa·s, besonders bevorzugt im Bereich von 100 bis 500 mPa·s liegen.

Mit Hilfe der erfindungsgemäßen Lösungsvermittler gelingt es, in Wasser schwerlösliche Komponenten, insbesondere Parfümöle, Vitamine oder UV-Lichtschutzfilter, in Formulierungen mit hohen Wassergehalten einzuarbeiten und klare Produkte zu erhalten.

Die Herstellung der ethoxylierten Fettalkohole der Komponente a) erfolgt großtechnisch durch basenkatalysierte Anlagerung von Ethylenoxid an die primäre Hydroxylfunktion der Alkohole.

Bei den Anlagerungsprodukten von Ethylenoxid an Triglyceride, die die Komponente b) bilden, handelt es sich um bekannte nichtionische Tenside. In Frage kommen beispielsweise Anlagerungsprodukte von durchschnittlich 20 bis 100 Mol, vorzugsweise 30 bis 80 Mol, besonders bevorzugt 35 bis 65 Mol, und insbesondere bevorzugt 40 Mol Ethylenoxid an Triglyceride, deren Acylreste sich von Fettsäuren mit 6 bis 22, vorzugsweise 12 bis 18 Kohlenstoffatomen ableiten. Die Triglyceride können synthetischer Natur sein. Vorzugsweise handelt es sich jedoch um natürliche, speziell pflanzliche Fette und Öle, die nach Raffination und Härtung mit Ethylenoxid umgesetzt werden. Die Ethoxylierung kann dabei durch Einschub von Ethylenoxid in die Carbonylestergruppe erfolgen. Leichter und daher auch bevorzugt ist jedoch die Anlagerung an im Molekül vorhandene, sekundäre Hydroxylgruppen, weshalb gehärtetes Ricinusöl (Castoröl) als Ausgangsstoff besonders bevorzugt ist. Aus anwendungstechnischer Sicht empfiehlt sich insbesondere der Einsatz von Anlagerungsprodukten von durchschnittlich 35 bis 65 Mol und vorzugsweise 40 Mol, Ethylenoxid an gehärtetes, d. h. hydriertes, Castoröl.

Die erfindungsgemäßen Lösungsvermittler können zur Herstellung von kosmetischen, dermatologischen oder pharmazeutischen Formulierungen, wie beispielsweise Haarshampoos, Haarlotionen, Schaumbädern, Duschbädern, Mund-und Zahnpflegemitteln, Cremes, Gelen, Lotionen, alkoholischen und wässrig/alkoholischen Lösungen, Emulsionen, Wachs/Fett-Massen, Stiftpräparaten, Pudern oder Salben dienen.

Weiterer Gegenstand der vorliegenden Erfindung ist daher auch die Verwendung eines erfindungsgemäßen Lösungsvermittlers zur Herstellung von kosmetischen, dermatologischen und pharmazeutischen Formulierungen.

Weiterer Gegenstand der vorliegenden Erfindung sind auch kosmetische, dermatologische oder pharmazeutische Formulierungen hergestellt unter Verwendung eines erfindungsgemäßen Lösungsvermittlers.

In diesen kosmetischen, dermatologischen oder pharmazeutischen Formulierungen sind die erfindungsgemäßen Lösungsvermittler vorzugsweise in Mengen von 0,1 bis 10,0 Gew.-%, bezogen auf die fertigen Formulierungen, enthalten.

Diese erfindungsgemäßen Formulierungen enthalten entweder keine Propylenoxid-haltigen Substanzen oder in dem Fall, dass sie Propylenoxid-haltige Substanzen enthalten, bezogen auf die fertigen Formulierungen, vorzugsweise 1,0 Gew.-% oder weniger, besonders bevorzugt 0,05 Gew.-% oder weniger, und insbesondere bevorzugt 0,01 Gew.-% oder weniger, an Propylenoxid-haltigen Substanzen.

Besonders bevorzugt enthalten die unter Verwendung der erfindungsgemäßen Lösungsvermittler hergestellten erfindungsgemäßen kosmetischen, dermatologischen oder pharmazeutischen Formulierungen
a1) C₁₁-Alkyl-O-(CH₂CH₂O)₈-H,
b1) ethoxyliertes hydriertes Castoröl mit 40 Ethylenoxid-Einheiten, und
c) Wasser und
d) entweder keine Propylenoxid-haltigen Substanzen oder in dem Fall, dass sie Propylenoxid-haltige Substanzen enthalten, bezogen auf die fertigen Formulierungen, 1,0 Gew.-% oder weniger, vorzugsweise 0,05 Gew.-% oder weniger und besonders bevorzugt 0,01 Gew.-% oder weniger, an Propylenoxid-haltigen Substanzen.

Da derartige kosmetische, dermatologische oder pharmazeutische Formulierungen auch anstelle der Verwendung der erfindungsgemäßen Lösungsvermittler unter Verwendung der in den erfindungsgemäßen Lösungsvermittlern enthaltenen Einzelkomponenten hergestellt werden können, betrifft ein weiterer Gegenstand der vorliegenden Erfindung auch kosmetische, dermatologische oder pharmazeutische Formulierungen, die
a1) C₁₁-Alkyl-O-(CH₂CH₂O)₈-H,
b1) ethoxyliertes hydriertes Castoröl mit 40 Ethylenoxid-Einheiten, und
c) Wasser enthalten und
d) entweder keine Propylenoxid-haltigen Substanzen enthalten oder in dem Fall, dass sie Propylenoxid-haltige Substanzen enthalten, bezogen auf die fertigen Formulierungen, 1,0 Gew.-% oder weniger, vorzugsweise 0,05 Gew.-% oder weniger und besonders bevorzugt 0,01 Gew.-% oder weniger, an Propylenoxid-haltigen Substanzen enthalten.

Vorzugsweise ist, bezogen auf die fertigen Formulierungen, der ethoxylierte Alkohol C₁₁-Alkyl-O-(CH₂CH₂O)₈-H der Komponente a1) von 0,05 bis 6,0 Gew.-% und das ethoxylierte hydrierte Castoröl mit 40 Ethylenoxid-Einheiten der Komponente b1) von 0,02 bis 5,0 Gew.-% in den erfindungsgemäßen Formulierungen enthalten.

Besonders bevorzugt ist, bezogen auf die fertigen Formulierungen, der ethoxylierte Alkohol C₁₁-Alkyl-O-(CH₂CH₂O)₈-H der Komponente a1) von 0,056 bis 5,6 Gew.-% und das ethoxylierte hydrierte Castoröl mit 40 Ethylenoxideinheiten der Komponente b1) von 0,03 bis 3,0 Gew.-% in den erfindungsgemäßen Formulierungen enthalten.

In einer insbesondere bevorzugten Ausführungsform der Erfindung enthalten die erfindungsgemäßen Formulierungen keine Popylenoxid-haltigen Substanzen.

Die erfindungsgemäßen Formulierungen enthalten, bezogen auf die fertigen Formulierungen, vorzugsweise eine Menge an Wasser von 30,0 Gew.-% oder mehr, besonders bevorzugt von 40,0 bis 95,0 Gew.-%, insbesondere bevorzugt von 50,0 bis 95,0 Gew.-% und außerordentlich bevorzugt von 60,0 bis 95,0 Gew.-%.

Weiterhin bevorzugt enthalten die erfindungsgemäßen Formulierungen in Wasser schwerlösliche Komponenten, insbesondere ausgewählt aus Parfümölen, Vitaminen und UV-Lichtschutzfiltern. Diese erfindungsgemäßen Formulierungen enthalten, bezogen auf die fertigen Formulierungen, vorzugsweise 0,01 bis 30,0 Gew.-%, besonders bevorzugt 0,01 bis 20,0 Gew.-%, insbesondere bevorzugt 0,05 bis 10,0 Gew.-% und außerordentlich bevorzugt 0,5 bis 5,0 Gew.-%, an in Wasser schwerlöslichen Komponenten, insbesondere an in Wasser schwerlöslichen Komponenten ausgewählt aus Parfümölen, Vitaminen und UV-Lichtschutzfiltern.

In einer besonders bevorzugten Ausführungsform der Erfindung enthalten die erfindungsgemäßen Formulierungen ein oder mehrere in Wasser schwerlösliche Parfümöle. Die in Wasser schwerlöslichen Parfümöle sind in den erfindungsgemäßen Formulierungen in Mengen von vorzugsweise 0,01 bis 20,0 Gew.-%, besonders bevorzugt 0,05 bis 10,0 Gew.-% und insbesondere bevorzugt 0,5 bis 5,0 Gew.-%, bezogen auf die fertigen erfindungsgemäßen Formulierungen, enthalten.

Als in Wasser schwerlösliche Parfümöle stehen Gemische aus natürlichen und synthetischen Riechstoffen zur Verfügung. Bevorzugte natürliche Riechstoffe sind Extrakte von Blüten (Rosen, Lilien, Lavendel, Kamille, Lindenblüte, Jasmin, Neroli, Ylang-Ylang), Stengeln und Blüten (Geranium, Patchouli, Petitgrain), Früchten (Anis, Nelken, Koriander, Kümmel, Wacholder, Mango, Pfirsich, Vanille), Fruchtschalen (Bergamotte, Zitronen, Orangen), Wurzeln (Macis, Angelica, Sellerie, Kardamon, Costus, Iris, Calmus), Hölzern (Pinien-, Sandel-, Gujak-, Zedern-, Rosenholz), Kräutern und Gräsern (Estragon, Lemongras, Salbei, Thymian, Rosmarin, Minze, Melisse, Zimtblätter), Nadeln und Zweigen (Fichte, Tanne, Kiefer, Latschen), Harzen und Balsamen (Galbanum, Elemi, Benzoe, Myrrhe, Olibanum, Opoponax). Als weiterhin bevorzugte Duft- bzw. Parfümöle können auch synthetische Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z. B. die lonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

In einer weiteren besonders bevorzugten Ausführungsform der Erfindung enthalten die erfindungsgemäßen Formulierungen ein oder mehrere in Wasser schwerlösliche Vitamine. Die in Wasser schwerlöslichen Vitamine sind in den erfindungsgemäßen Formulierungen in Mengen von vorzugsweise 0,001 bis 10,0 Gew.-%, besonders bevorzugt 0,01 bis 10,0 Gew.-%, insbesondere bevorzugt 0,05 bis 10,0 Gew.-% und außerordentlich bevorzugt 0,5 bis 5,0 Gew.-%, bezogen auf die fertigen erfindungsgemäßen Formulierungen, enthalten.

Als in Wasser schwerlösliche Vitamine kommen vorzugsweise zum Einsatz Vitamin A, Vitamin-A-Derivate, Carotine oder deren Derivate, Ascorbinsäure (Vitamin C), sowie Tocopherol (Vitamin E) und/oder dessen Derivate.

In einer weiteren besonders bevorzugten Ausführungsform der Erfindung enthalten die erfindungsgemäßen Formulierungen ein oder mehrere in Wasser schwerlösliche UV-Filter. Die in Wasser schwerlöslichen UV-Filter sind in den erfindungsgemäßen Formulierungen in Mengen von vorzugsweise 0,01 bis 20,0 Gew.-%, besonders bevorzugt 0,05 bis 10,0 Gew.-% und insbesondere bevorzugt 0,5 bis 5,0 Gew.-%, bezogen auf die fertigen erfindungsgemäßen Formulierungen, enthalten.

Als in Wasser schwerlösliche UV-Filter kommen vorzugsweise in Betracht 4-Aminobenzoesäure;
3-(4'-Trimethylammonium)benzyliden-boran-2-on-methylsulfat;
3,3,5-Trimethyl-cyclohexylsalicylat; 2-Hydroxy-4-methoxybenzophenon;
2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure) und ihre Salze;
1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester);
Polymer von N-[2(und 4)-(2-oxoborn-3-ylidenmethyl)benzyl]-acrylamid; 4-Methoxyzimtsäure-2-ethyl-hexylester; ethoxyliertes Ethyl-4-amino-benzoat; 4-Methoxyzimtsäure-isoamylester; 2,4,6-Tris-[p-(2-ethylhexyloxycarbonyl)anilino]-1,3,5-triazin;
2-(2H-benzotriazol-2-yl)-4-methyl-6-(2-methyl-3-(1,3,3,3-tetramethyl-1-(trimethylsilyloxy)-disiloxanyl)propyl)phenol;
4,4'-[(6-[4-((1,1-dimethylethyl)-amino-carbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester;
4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat.

Die erfindungsgemäßen kosmetischen, dermatologischen oder pharmazeutischen Formulierungen können als weitere Hilfs- und Zusatzstoffe Tenside, Emulgatoren, kationische Polymere, Verdicker, Filmbildner, antimikrobielle Wirkstoffe, Adstringentien, Antioxidantien, UV-Lichtschutzfilter, Pigmente/Mikropigmente, Gelierungsmittel, sowie weitere in der Kosmetik, Dermatologie oder Pharmazie gebräuchliche Zusätze, wie z. B. Überfettungsmittel, feuchtigkeitsspendende Mittel, Silicone, Stabilisatoren, Konditioniermittel, Glycerin, Konservierungsmittel, Perlglanzmittel, Farbstoffe, Enzyminhibitoren, Lösungsmittel, Hydrotrope, Trübungsmittel, Fettalkohole, Stoffe mit keratolytischer und keratoplastischer Wirkung, Antischuppenmittel, biogene Wirkstoffe (Lokalanästhetika, Antibiotika, Antiphlogistik, Antiallergica, Corticosteroide, Sebostatika), Vitamine, Bisabolol^{®}, Allantoin, Phytantriol^{®}, Panthenol^{®}, AHA-Säuren, Pflanzenextrakte, beispielsweise Aloe Vera und Proteine enthalten.

Sofern als weitere Hilfs- und Zusatzstoffe für die erfindungsgemäßen kosmetischen, dermatologischen oder pharmazeutischen Formulierungen im Folgenden Substanzen genannt sind, die bereits von den erfindungsgemäßen Lösungsvermittlern umfasst sind, und zwar entweder explizit als Einzelverbindungen oder in der Form von generischen Ausdrücken, ist dies dadurch begründet, dass diese Substanzen prinzipiell zusätzlich zu den erfindungsgemäßen Lösungsvermittlern zur Herstellung der erfindungsgemäßen Formulierungen verwendet werden können.

In einer bevorzugten Ausführungsform der Erfindung umfasst die Gruppe der im Folgenden genannten weiteren Hilfs- und Zusatzstoffe die Verbindungen der Komponenten a) und b) der erfindungsgemäßen Lösungsvermittler jedoch nicht. In einer weiteren bevorzugten Ausführungsform der Erfindung umfasst die Gruppe der im Folgenden genannten weiteren Hilfs- und Zusatzstoffe lediglich die Substanzen C₁₁-Alkyl-O-(CH₂CH₂O)₈-H und ethoxyliertes hydriertes Castoröl mit 40 Ethylenoxid-Einheiten nicht.

Als anionische waschaktive Substanzen bzw. Tenside seien vorzugsweise genannt: C₁₀-C₂₀-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäure-methyltauride, Fettsäuresarkosinate, Sulforicinoleate, Amphoacetate- oder -glycinate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie der analogen Alkylammonium-Salze.

Die Menge der anionischen Tenside in den erfindungsgemäßen Formulierungen beträgt bevorzugt 1 bis 30 Gew.-%, besonders bevorzugt 5 bis 25 Gew.-%, und insbesondere bevorzugt 10 bis 22 Gew.-%, bezogen auf die fertigen Formulierungen.

Geeignete kationische Tenside sind beispielsweise quartäre Ammonium-Salze wie Di-(C₁₀-C₂₄-Alkyl)-dimethyl-ammonium-chlorid oder -bromid, vorzugsweise Di-(C₁₂-C₁₈-Alkyl)-dimethyl-ammonium-chlorid oder -bromid; C₁₀-C₂₄-Alkyl-dimethyl-ethylammonium-chlorid oder -bromid; C₁₀-C₂₄-Alkyl-trimethyl-ammonium-chlorid oder -bromid, vorzugsweise Cetyl-trimethyl-ammonium-chlorid oder -bromid und C₂₀-C₂₂-Alkyl-trimethyl-ammonium-chlorid oder -bromid; C₁₀-C₂₄-Alkyl-dimethylbenzyl-ammonium-chlorid oder -bromid, vorzugsweise C₁₂-C₁₈-Alkyl-dimethylbenzyl-ammonium-chlorid; N-(C₁₀-C₁₈-Alkyl)-pyridinium-chlorid oder -bromid, vorzugsweise N-(C₁₂-C₁₆-Alkyl)-pyridinium-chlorid oder -bromid; N-(C₁₀-C₁₈-Alkyl)-isochinolinium-chlorid, -bromid oder -monoalkylsulfat; N-(C₁₂-C₁₈-Alkylpolyoylaminoformylmethyl)-pyridinium-chlorid; N-(C₁₂-C₁₈-Alkyl)-N-methyl-morpholinium-chlorid, -bromid oder -monoalkylsulfat; N-(C₁₂-C₁₈-Alkyl)-N-ethyl-morpholinium-chlorid, -bromid oder -monoalkylsulfat; C₁₆-C₁₈-Alkyl-pentaoxethyl-ammonium-chlorid; Diisobutylphenoxyethoxyethyldimethylbenzylammonium-chlorid; Salze des N,N-Diethylamino-ethylstearylamids und -oleylamids mit Salzsäure, Essigsäure, Milchsäure, Zitronensäure, Phosphorsäure; N-Acyl-aminoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Die Menge der kationischen Tenside in den erfindungsgemäßen Formulierungen beträgt bevorzugt 0,1 bis 10 Gew.-%, besonders bevorzugt 0,2 bis 7 Gew.-%, und insbesondere bevorzugt 0,5 bis 5 Gew.-%, bezogen auf die fertigen Formulierungen.

Als nichtionische Tenside, die als waschaktive Substanzen eingesetzt werden können, kommen vorzugsweise in Betracht Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäureamidpolyethylenglykole; N-Alkyl-, N-Alkoxypolyhydroxyfettsäureamid, insbesondere Fettsäure-N-methylglucamide, Saccharoseester; Polyglykolether, Alkylpolyglycoside, Phosphorsäureester (Mono-, Di- und Triphosphorsäureester ethoxyliert und nicht-ethoxyliert).

Die Menge der nichtionischen Tenside in den erfindungsgemäßen Formulierungen (z. B. im Falle von Rinse-off-Produkten) liegt bevorzugt im Bereich von 1 bis 20 Gew.-%, besonders bevorzugt 2 bis 10 Gew.-%, und insbesondere bevorzugt 3 bis 7 Gew.-%, bezogen auf die fertigen Formulierungen.

Bevorzugte Amphotenside sind: N-(C₁₂-C₁₈-Alkyl)-β-aminopropionate und N-(C₁₂-C₁₈-Alkyl)-β-iminodipropionate als Alkali- und Mono-, Di- und Trialkylammonium-Salze;
N-Acylaminoalkyl-N,N-dimethyl-acetobetain, vorzugsweise N-(C₈-C₁₈-Acyl)aminopropyl-N,N-dimethylacetobetain; C₁₂-C₁₈-Alkyl-dimethyl-sulfopropyl-betain; Amphotenside auf Basis Imidazolin (Handelsname: Miranol^{®}, Steinapon^{®}), vorzugsweise das Natrium-Salz des 1-(β-Carboxy-methyloxyethyl)-1-(carboxymethyl)-2-lauryl-imidazoliniums; Aminoxide, z. B.
C₁₂-C₁₈-Alkyldimethylaminoxid, Fettsäureamidoalkyl-dimethylaminoxid.

Die Menge der amphoteren Tenside in den erfindungsgemäßen Formulierungen beträgt bevorzugt 0,5 bis 20 Gew.-% und besonders bevorzugt 1 bis 10 Gew.-%, bezogen auf die fertigen Formulierungen.

Des Weiteren können in den erfindungsgemäßen Formulierungen schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den erfindungsgemäßen Formulierungen sind Alkylethersulfate, Alkylsulfate, insbesondere Laurylsulfat, Alkylbetaine, Alkylamidopropylbetaine, insbesondere Cocoamidopropylbetain, Amphoacetate, Acylglutamate, insbesondere Natriumcocoylglutamat, Alkylethersulfosuccinate, insbesondere Di-natriumlaureth-sulfosuccinat, und Cocosfettsäurediethanolamid.

Die Gesamtmenge der in den erfindungsgemäßen Formulierungen eingesetzten Tenside beträgt vorzugsweise 1 bis 70 Gew.-%, besonders bevorzugt 10 bis 40 Gew.-%, und insbesondere bevorzugt 12 bis 35 Gew.-%, bezogen auf die fertigen Formulierungen.

Als Emulsionen vorliegende erfindungsgemäße Formulierungen können ohne weiteren Emulgator erzeugt werden oder auch einen oder mehrere Emulgatoren enthalten. Diese Emulgatoren können gewählt werden aus der Gruppe der nichtionischen, anionischen, kationischen oder amphoteren Emulgatoren.

Als nichtionogene Co-Emulgatoren kommen vorzugsweise in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z. B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Weiterhin können natürlich vorkommende Emulgatoren, unter denen Bienenwachs, Wollwachs, Lecithin und Sterole bevorzugt sind, verwendet werden.

Vorzugsweise sind Fettalkoholethoxylate gewählt aus der Gruppe der ethoxylierten Stearylalkohole, Cetylalkohole, Oleylalkohole, Laurylalkohole, Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol(18)stearylether, Polyethylenglycol(19)stearylether, Polyethylenglycol(20)stearylether, Polyethylenglycol(12)isostearylether, Polyethylenglycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethylenglycol(13)cetylether, Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetylether, Polyethylenglycol(15)isocetylether, Polyethylenglycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol(14)oleylether, Polyethylenglycol(15)oleylether, Polyethylenglycol(12)laurylether, Polyethylenglycol(12)isolaurylether, Polyethylenglycol(13)cetylstearylether, Polyethylenglycol(14)cetylstearylether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether, Polyethylenglycol(20)cetylstearylether.

Vorzugsweise sind Fettsäureethoxylate gewählt aus der Gruppe der ethoxylierten Stearate, Isostearate und Oleate, insbesondere Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat, Polyethylenglykol(6)glycerylcaprat/caprinat, Polyethylenglykol(20)glyceryloleat, Polyethylenglykol(20)glycerylisostearat und Polyethylenglykol(18)glyceryloleat/cocoat zu wählen.

Unter den Sorbitanestern eignen sich besonders Polyethylenglykol(20)sorbitanmonolaurat, Polyethylenglykol(20)sorbitanmonostearat, Polyethylenglykol(20)sorbitanmonoisostearat, Polyethylenglykol(20)sorbitanmonopalmitat, Polyethylenglykol(20)sorbitanmonooleat.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglykolester gesättigter und/oder ungesättigter, verzweigter und/oder unverzweigter Alkancarbonsäuren einer Kettenlänge von 8 bis 24, insbesondere 12 bis 18 C-Atomen, sowie Sorbitanester gesättigter und/oder ungesättigter, verzweigter und/oder unverzweigter Alkancarbonsäuren einer Kettenlänge von 8 bis 24, insbesondere 12 bis 18 C-Atomen. Insbesondere vorteilhafte W/O-Emulgatoren sind Glycerylmonostearat, Glycerylmonoisostearat, Glycerylmonomyristat, Glycerylmonooleat, Glycerylmonolaurat, Glycerylmonocaprylat, Glycerylmonocaprinat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol oder Polyethylenglycol(2)stearylether.

Die Menge des oder der in den erfindungsgemäßen Formulierungen enthaltenen Emulgators oder Emulgatoren, beträgt vorzugsweise 0,1 bis 20 Gew.-%, besonders bevorzugt 0,5 bis 15 Gew.-%, und insbesondere bevorzugt 1 bis 10 Gew.-%, bezogen auf die fertigen Formulierungen.

Als kationische Polymere eignen sich vorzugsweise die unter der INCI-Bezeichnung "Polyquaternium" bekannten Verbindungen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium 37&mineral oil&PPG trideceth (Salcare^{®} SC95), PVP-dimethylaminoethylmethacrylat-Copolymer, Guarhydroxypropyltriammoniumchloride, sowie Calciumalginat und Ammoniumalginat.

Des weiteren können vorzugsweise eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxypropyldiethylentriamin; Polyaminopolyamid und kationische Chitinderivate, wie beispielsweise Chitosan.

Die Menge an kationischen Polymeren in den erfindungsgemäßen Formulierungen kann vorzugsweise im Bereich von 0,1 bis 10 Gew.-%, besonders bevorzugt im Bereich von 0,2 bis 5 Gew.-%, und insbesondere bevorzugt im Bereich von 0,5 bis 2,5 Gew.-% liegen, bezogen auf die fertigen Formulierungen.

Die gewünschte Viskosität der Formulierungen kann durch Zugabe von Verdickungsmitteln eingestellt werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester.

Als synthetische Polymere kommen verschiedene Materialien zum Einsatz, vorzugsweise Polyvinylalkohole, Polyacrylamide, Polyvinylamide, Polysulfonsäuren, insbesondere Copolymerisate auf Basis von Ammoniumsalzen von Acrylamidoalkylsulfonsäuren und cyclischen N-Vinylcarbonsäureamiden bzw. cyclischen und linearen N-Vinylcarbonsäureamiden oder auch hydrophob modifizierte Acrylamidoalkylsulfonsäure-Copolymerisate, Polyacrylsäure, Polyacrylsäurederivate, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den oben genannten Verbindungen, einschließlich ihrer verschiedenen Salze und Ester. Diese Polymere können wahlweise vernetzt oder unvernetzt sein.

Insbesondere für Formulierungen auf Ölbasis besonders geeignete Verdicker sind Dextrinester, beispielsweise Dextrinpalmitat, aber auch Fettsäureseifen, Fettalkohole und Silikonwachse, beispielsweise Alkylmethicone, SilCare® 41 M40, SilCare® 41 M50, SilCare® 41 M65, SilCare® 41 M70 oder SilCare® 41 M80.

Bevorzugte Filmbildner sind je nach Anwendungszweck Salze der Phenylbenzimidazolsulfonsäure, wasserlösliche Polyurethane, beispielsweise C₁₀-Polycarbamylpolyglycerylester, Polyvinylalkohol, Polyvinylpyrrolidoncopolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carboxyvinyl-Polymere, wie Carbomere und deren Salze, Polysaccharide, beispielsweise Polydextrose und Glucan, Vinylacetat/Crotonat, beispielsweise unter dem Handelsnamen Aristoflex^{®} A 60 (Clariant) erhältlich, sowie polymere Aminoxide, beispielsweise unter den Handelsnamen Diaformer Z-711, 712, 731, 751 erhältliche Vertreter.

Bevorzugt geeignet als antimikrobielle Wirkstoffe sind Cetyltrimethylammoniumchlorid, Cetylpyridiniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol und Kombinationen dieser Wirksubstanzen.

Die erfindungsgemäßen Formulierungen enthalten die antimikrobiellen Mittel bevorzugt in Mengen bis 50 Gew.-%, besonders bevorzugt in Mengen von 0,01 bis 10 Gew.-%, und insbesondere bevorzugt in Mengen von 0,1 bis 10 Gew.-%, bezogen auf die fertigen Formulierungen.

Bevorzugte Adstringentien sind Oxide, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink.

Die erfindungsgemäßen Formulierungen enthalten die adstringenden Wirkstoffe bevorzugt in Mengen von 0 bis 50 Gew.-%, besonders bevorzugt in Mengen von 0,01 bis 10 Gew.-% und insbesondere bevorzugt in Mengen von 0,1 bis 10 Gew.-%, bezogen auf die fertigen Formulierungen.

Vorteilhafte erfindungsgemäße Formulierungen enthalten ein oder mehrere Antioxidantien.

Als günstige, aber dennoch fakultativ zu verwendende Antioxidantien können alle für kosmetische, dermatologische und/oder pharmazeutische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. (α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Penta-, Hexa-, Heptathioninsulfoximin) in sehr geringen verträglichen Dosierungen (z. B. pmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄), Selen und dessen Derivate (z. B. Selenmethionin), Stilbene und deren Derivate (z.B. Stilbenoxid, Trans-Stilbenoxid), Superoxid-Dismutase und die erfindungsgemäß geeigneten Derivate (Salze, Ester, Ether, Zucker, Nukleotide, Nukleoside, Peptide und Lipide) dieser genannten Stoffe.

Besonders vorteilhaft im Sinne der vorliegenden Erfindung können wasserlösliche Antioxidantien eingesetzt werden.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge der Antioxidantien (eine oder mehrere Verbindungen) in den erfindungsgemäßen Formulierungen beträgt vorzugsweise 0,001 bis 30 Gew.-%, besonders bevorzugt 0,05 bis 20 Gew.-%, und insbesondere 1 bis 10 Gew.-%, bezogen auf die fertigen Formulierungen.

Als Pigmente/Mikropigmente können vorzugsweise mikrofeines Titandioxid, Glimmer-Titanoxid, Eisenoxide, Glimmer-Eisenoxid, Zinkoxid, Siliziumoxide, Ultramarinblau, Chromoxide, eingesetzt werden.

Als Gelierungsmittel eignen sich alle oberflächenaktiven Stoffe, die in der flüssigen Phase gelöst eine Netzwerkstruktur ausbilden und so die flüssige Phase verfestigen. Geeignete Geliermittel sind z. B. in WO 98/58625 genannt.

Bevorzugte Gelierungsmittel sind Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher.

Bevorzugt enthalten die erfindungsgemäßen Formulierungen 0,01 bis 20 Gew.-%, besonders bevorzugt 0,1 bis 10 Gew.-%, insbesondere bevorzugt 1 bis 8 Gew.-% und außerordentlich bevorzugt 3 bis 7 Gew.-% an Geliermitteln, bezogen auf die fertigen Formulierungen.

Weitere Zusatzstoffe können Siliconverbindungen sein, vorzugsweise Dimethylpolysiloxan, Methyl-phenylpolysiloxane, cyclische Silicone sowie amino-, fettsäure-, alkohol-, polyether-, epoxy-, fluor- und/oder alkylmodifizierte Siliconverbindungen, beispielsweise Alkylsilicone SilCare^{®} Silicone 41M10, SilCare^{®} Silicone 41M15, SilCare^{®} Silicone 41 M20, SilCare^{®} Silicone 41 M30 (Clariant), Alkyltrimethicone SilCare^{®} 31 M30, SilCare^{®} 31 M40, SilCare® 31 M50, SilCare^{®} 31 M60 (Clariant), Phenyltrimethicone SilCare^{®} 15M30, SilCare^{®} 15M40, SilCare^{®} 15M50, SilCare^{®} 15M60 (Clariant), Polyalkylarylsiloxane und Polyethersiloxan-Copolymere.

Die erfindungsgemäßen Formulierungen können die oben genannten Siliconverbindungen vorzugsweise in den Mengen von 0,1 bis 20 Gew.-%, besonders bevorzugt 0,2 bis 15 Gew.-%, insbesondere bevorzugt 0,5 bis 10 Gew.-%, bezogen auf die fertigen Formulierungen, enthalten.

Als Trägermaterialien in Betracht kommen vorzugsweise pflanzliche Öle, natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und Cellulose-Derivate.

Als fungizide Wirkstoffe können vorzugsweise Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Zn-Pyrithion und Octopirox^{®} in den Mengen von 0,05 bis 5 Gew.-%, bevorzugt 0,1 bis 3 Gew.-%, und besonders bevorzugt 0,2 bis 2 Gew.-%, bezogen auf die fertigen Formulierungen, eingesetzt werden.

Die erfindungsgemäßen Formulierungen können vorteilhaft mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen, abgemischt werden.

Als perlglanzgebende Verbindungen bevorzugt sind Fettsäuremonoalkanolamide, Fettsäuredialkanolamide, Monoester oder Diester von Alkylenglykol, insbesondere des Ethylenglykols und/oder Propylenglykols oder dessen Oligomere mit höheren Fettsäuren, z. B. Palmitinsäure, Stearinsäure oder Behensäure oder Mischungen davon, Mono- oder Diester von Alkylenglykolen mit Fettsäuren, Fettsäuren und deren Metallsalze, Monoester oder Polyester von Glycerin mit Carbonsäuren und Ketosulfone verschiedener Art. In den erfindungsgemäßen Formulierungen sind als perlglanzgebende Komponente besonders bevorzugt Ethylenglykoldistearat und Polyethylenglykoldistearat mit 3 Glykoleinheiten.

Als feuchtigkeitsspendende Substanz stehen vorzugsweise Isopropylpalmitat, Glycerin und/oder Sorbitol zu Verfügung, die bevorzugt in den Mengen von 0,1 bis 50 Gew.-% eingesetzt werden, bezogen auf die fertigen erfindungsgemäßen Formulierungen.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide verwendet werden.

Als Konservierungsmittel in Betracht kommen vorzugsweise Phenoxyethanol, Parabene, Pentandiol oder Sorbinsäure. Sie werden vorzugsweise in Mengen von 0,001 bis 5 Gew.-%, besonders bevorzugt von 0,01 bis 3 Gew.-%, und insbesondere bevorzugt von 0,1 bis 2 Gew.-%, bezogen auf die fertigen erfindungsgemäßen Formulierungen, eingesetzt.

Als Enzyminhibitoren sind beispielsweise Esteraseinhibitoren geeignet. Hierbei handelt es sich vorzugsweise um Trialkylcitrate, wie Trimethylcitrat, Tripropylcitrat, Triisopropylcitrat, Tributylcitrat und insbesondere Triethylcitrat (Hydagen TM CAT, Henkel KGaA). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate.

Als Farbstoffe können die für kosmetische, dermatologische und pharmazeutische Zwecke geeigneten und zugelassenen Substanzen verwendet werden.

Der Gesamtanteil der Hilfs- und Zusatzstoffe kann 1 bis 50 Gew.-% und vorzugsweise 5 bis 40 Gew.-%, bezogen auf die fertigen erfindungsgemäßen Formulierungen, betragen. Die Herstellung der erfindungsgemäßen Formulierungen kann durch übliche Kalt- oder Heißprozesse erfolgen.

Als Säuren oder Laugen zur pH-Wert Einstellung werden vorzugsweise Mineralsäuren, insbesondere HCl, anorganische Basen, insbesondere NaOH oder KOH, und organische Säuren, insbesondere Zitronensäure, verwendet.

Die erfindungsgemäßen Formulierungen sind vorzugsweise auf einen pH-Wert im Bereich 2 bis 12 und besonders bevorzugt auf einen pH-Wert im Bereich 3 bis 8 eingestellt.

In einer weiteren besonders bevorzugten Ausführungsform der Erfindung handelt es sich bei den erfindungsgemäßen Formulierungen um Formulierungen mit einem klaren Aussehen.

Die nachfolgenden Beispiele und Anwendungen sollen die Erfindung näher erläutern, ohne sie jedoch darauf zu beschränken (bei allen Prozentangaben handelt es sich um Gewichtsprozent; Gew.-%).

### Beispiele

### Beispiele 1 bis 4: Lösungsvermittler

| | | |
|---|---|---|
| 1 | Genapol^{®} UD 088 | 50 Gew.-% |
| | Emulsogen^{®} HCO 040 | 50 Gew.-% |
| | | |
| 2 | Genapol^{®} UD 088 | 50 Gew.-% |
| | Emulsogen^{®} HCO 060 | 50 Gew.-% |
| | | |
| 3 | Genapol^{®} UD 088 | 70 Gew.-% |
| | Emulsogen^{®} HCO 040 | 30 Gew.-% |
| | | |
| 4 | Genapol^{®} UD 110 | 44 Gew.-% |
| | Emulsogen^{®} HCO 060 | 44 Gew.-% |
| | Wasser | 12 Gew.-% |

Die Beispiele 1 bis 4 sind erfindungsgemäß. Beispiele 1 bis 3 sind beispielsweise deshalb erfindungsgemäß, weil Genapol^{®} UD 088 20 Gew.-% Wasser enthält.

### Formulierungsbeispiele 5 bis 7: After Shave Gel

### Zusammensetzung:

| | | |
|---|---|---|
| A | Lösungsvermittler gemäß Beispiel 1, 2 oder 3 | 1,5 % |
| B | Vitamine E | 0,2 % |
| | Menthol | 0,2 % |
| C | Alkohol | 30,0 % |
| D | Wasser | ad 100 % |
| | Allantoin | 0,2 % |
| | Polyglykol 400 | 3,0 % |
| | Polyglykol 35000 P | 1,0 % |
| E | Aristoflex^{®} AVC | 1,0 % |

### Herstellung:

- I: Die Komponenten A und B werden zusammen vorgelegt und 5 Minuten miteinander verrührt.
- II: Komponente C zu Mischung I geben und ebenfalls 5 Minuten rühren.
- III: Komponenten D zusammen einwiegen und anschließend zu Mischung II geben, nachfolgend weitere 5 Minuten rühren.
- IV: Komponente E zu Mischung III geben und bis zur vollständigen Quellung der Komponente E rühren (mindestens 2 Stunden).

Die Formulierungsbeispiele 5 bis 7 sind erfindungsgemäß. Zum Vergleich wurden die Formulierungsbeispiele V1 bis V3 hergestellt. Deren Zusammensetzungen entsprechen denen der Formulierungsbeispiele 5 bis 7, wobei jedoch anstelle der Lösungsvermittler gemäß Beispiel 1, 2 oder 3 Emulsogen^{®} HCO 040 (V1), Genapol^{®} UD 080 (V2) und Genapol^{®} UD 088 (V3) eingesetzt wurde. Der Austausch erfolgte, bezogen auf das Gewicht, in einem Verhältnis von 1:1.

Zur Beurteilung des Leistungsvermögens der erfindungsgemäßen Lösungsvermittler in klaren Formulierungen wurde die Transparenz der Formulierungen mittels NTU-Werten ermittelt. NTU ist die Abkürzung für "Nephelometric Turbidity Unit". Die Kalibriereinheit für Trübungsmessungen im Streulichtverfahren bei 90° wurde gemäß den Vorschriften der US-EPA (Environmental Protection Agency) angewendet, wobei der Standard auf Formazin-Lösung basiert.

Die Nephelometric Turbidity Unit (Nephelometrischer Trübungswert; NTU) ist eine in der Wasseraufbereitung verwendete Einheit für Trübungsmessungen in Flüssigkeiten. Sie ist die Einheit einer mit einem kalibrierten Nephelometer gemessenen Trübung einer Flüssigkeit.

**Tabelle 1: Transparenz der After Shave Gele gemäß den Formulierungen 5 bis 7**

| Formulierungsbeispiele | Verwendeter Lösungsvermittler | Transparenz der Gele (NTU) |
|---|---|---|
| 5 (Erfindung) | Beispiel 1 | 27 |
| 6 (Erfindung) | Beispiel 2 | 16 |
| 7 (Erfindung) | Beispiel 3 | 21 |
| V 1 (Vergleich) | Emulsogen^{®} HCO 040 | 36 |
| V 2 (Vergleich) | Genapol^{®} UD 080 | 3042 |
| V 3 (Vergleich) | Genapol^{®} UD 088 | 3970 |

### Formulierungsbeispiele 8 bis 10: Olivenölduschgel

### Zusammensetzung:

| | |
|---|---|
| Lösungsvermittler gemäß Beispiel 1, 2 oder 3 | 2,50 % |
| Olivenöl | 0,25 % |
| Genagen^{®} 3 SB | 40,00 % |
| Wasser | ad 100 % |
| Genapol^{®} DAT | 7,00 % |
| Phenonip^{®} | 0,50 % |

### Herstellung:

Die einzelnen Komponenten werden nacheinander eingewogen und unter Rühren miteinander vermischt.

Die Formulierungsbeispiele 8 bis 10 sind erfindungsgemäß. Das Vergleichsbeispiel V4 entspricht der Zusammensetzung der Formulierungsbeispiele 8 bis 10, wobei jedoch anstelle der Lösungsvermittler gemäß Beispiel 1, 2 oder 3 Emulsogen^{®} HCO 040 eingesetzt wurde. Der Austausch erfolgte, bezogen auf das Gewicht, in einem Verhältnis von 1 : 1.

**Tabelle 2: Transparenz der Duschgele gemäß den Formulierungen 8 bis 10**

| Formulierungsbeispiele | Verwendeter Lösungsvermittler | Transparenz des Duschgels (NTU) |
|---|---|---|
| 8 (Erfindung) | Beispiel 1 | 25 |
| 9 (Erfindung) | Beispiel 2 | 11 |
| 10 (Erfindung) | Beispiel 3 | 21 |
| V 4 (Vergleich) | Emulsogen^{®} HCO 040 | 43 |

### Formulierungsbeispiele 11 bis 14: Dauerwellenformulierung mit Parfümöl

### Zusammensetzung:

| | | |
|---|---|---|
| A | Wasser | ad 100 % |
| | | |
| B | Ammoniaklösung (25%) | 8,2 % |
| | Thioglycolsäure (80%) | 10,0 % |
| | Ammonium hydrogen carbonat | 1,5 % |
| | Genamin^{®} PDAC | 1,5 % |
| | | |
| C | Parfümöl | 0,8 % |
| | Lösungsvermittler gemäß Beispiel 1, 2, 3 oder 4 | 0,8 % |

### Herstellung:

- I: Die Komponenten B werden unter Rühren eine nach der anderen zur Komponente A gegeben.
- II: Die Komponenten C werden zusammen zur Mischung I geben und dabei alles miteinander verrührt.

Die Formulierungsbeispiele 11 bis 14 sind erfindungsgemäß. Beispiel V 5 dient zum Vergleich. Es entspricht der Zusammensetzung der Formulierungsbeispiele 11 bis 14, wobei jedoch anstelle der Lösungsvermittler gemäß Beispiel 1, 2, 3 oder 4 Emulsogen^{®} HCO 040 eingesetzt wurde. Der Austausch erfolgte, bezogen auf das Gewicht, in einem Verhältnis von 1 : 1.

**Tabelle 3: Transparenz der Dauerwellenformulierungen 11 bis 14**

| Formulierungsbeispiele | Verwendeter Lösungsvermittler | Transparenz der Dauerwellenformulierungen (NTU) |
|---|---|---|
| 11 (Erfindung) | Beispiel 1 | 20 |
| 12 (Erfindung) | Beispiel 2 | 17 |
| 13 (Erfindung) | Beispiel 3 | 19 |
| 14 (Erfindung) | Beispiel 4 | 24 |
| V 5 (Vergleich) | Emulsogen^{®} HCO 040 | 351 |

### Beispiele 15 bis 26 : Lösungsvermögen von Einzelsubstanzen (Rosmarinöl, Vanilia und Menthol) in wässriger Lösung

### Zusammensetzung der Testmischungen

| | |
|---|---|
| Testsubstanz | x % |
| Lösungsvermittler | 10 % |
| Wasser | ad 100 % |

### Vorgehensweise:

Zur Überprüfung des Lösungsvermögens wurden verschiedenen Mengen x der Testsubstanzen mit Lösungsvermittler bei 800 Umdrehungen 5 Minuten verrührt und anschließend die entsprechende Menge an 65 °C heißem Wasser zugegeben. Nach 5 Minuten weiteren Rührens wird die Klarheit der Lösung bewertet.

### Beispiele 15 bis 26 sind erfindungsgemäß. Die Beispiele V 6 bis V 8 dienen zum Vergleich.

**Tabelle 4: Lösungsvermögen (Mengenangaben in Gew.-%)**

| Formulierungsbeispiele | Verwendeter Lösungsvermittler [10 %] | Testsubstanz | Einsatzmenge der Testsubstanz bis zur Trübung der Testmischung [x %] |
|---|---|---|---|
| 15 (Erfindung) | Beispiel 1 | Rosmarinöl | 3 |
| 16 (Erfindung) | Beispiel 2 | Rosmarinöl | 3 |
| 17 (Erfindung) | Beispiel 3 | Rosmarinöl | 3 |
| 18 (Erfindung) | Beispiel 4 | Rosmarinöl | 3 |
| V 6 (Vergleich) | Genapol^{®} UD 080 | Rosmarinöl | 1 |
| 19 (Erfindung) | Beispiel 1 | Vanilia | 6 |
| 20 (Erfindung) | Beispiel 2 | Vanilia | 6 |
| 21 (Erfindung) | Beispiel 3 | Vanilia | 7 |
| 22 (Erfindung) | Beispiel 4 | Vanilia | 6 |
| V 7 (Vergleich) | Emulsogen^{®} HCO 040 | Vanilia | 5 |
| 23 (Erfindung) | Beispiel 1 | Menthol | 3 |
| 24 (Erfindung) | Beispiel 2 | Menthol | 4 |
| 25 (Erfindung) | Beispiel 3 | Menthol | 4 |
| 26 (Erfindung) | Beispiel 4 | Menthol | 4 |
| V 8 (Vergleich) | Emulsogen^{®} HCO 040 | Menthol | 1 |

### Chemische Bezeichnung der eingesetzten Handelsprodukte:

- Aristoflex^{®} AVC: Ammonium Acryloyldimethyltaurate/VP Copolymer
- Emulsogen^{®} HCO 040: hydriertes Castoröl mit 40 EO
- Emulsogen^{®} HCO 060: hydriertes Castoröl mit 60 EO
- Genagen^{®} 3 SB: Mischung aus Cocobetain, Natrium Methylcocoyltaurate und Natrium Cocoylisethionate
- Genamin^{®} PDAC: Polydiallyldimethylammonium chlorid
- Genapol^{®} DAT: Stearylester von Polyglycol ethoxyliert mit 150 EO
- Genapol^{®} UD 080: Gemisch aus linearem und verzeigtem C₁₁-Oxoalkoholpolyglykolether mit 8 EO
(Gewichtsverhältnis linear : verzweigt = 50 : 50)
- Genapol^{®} UD 088: Gemisch aus linearem und verzeigtem C₁₁-Oxoalkoholpolyglykolether mit 8 EO (80 Gew.-%) und
Wasser (20 Gew.-%)
(Gewichtsverhältnis linear : verzweigt = 50 : 50)
- Genapol^{®} UD 110: Gemisch aus linearem und verzeigtem
C₁₁-Oxoalkoholpolyglykolether mit 11 EO
(Gewichtsverhältnis linear : verzweigt = 50 : 50)
- Phenonip^{®}: Konservierungsmittelblend aus Phenoxyethanol, Methylparaben, Ethylparaben, Butylparaben, Propylparaben, Isobutylparaben
- Polyglykol 400: Polyethylenglykol mit einem mittleren
Molekulargewicht von 400
- Polyglykol 35000 P: Polyethylenglykol mit einem mittleren
Molekulargewicht von 350000

"EO" bedeutet eine Struktureinheit abgeleitet von Ethylenoxid, d.h. eine Struktureinheit -CH₂CH₂O-.

Die eingesetzten Handelsprodukte werden von Clariant vertrieben.

## Patentansprüche

1. Lösungsvermittler enthaltend
a) ein oder mehrere ethoxylierte Fettalkohole gemäß der Formel (I) 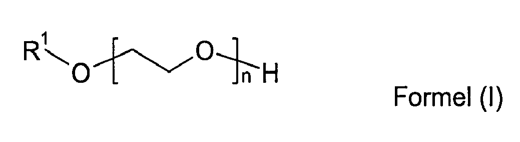 worin R¹ für einen linearen oder verzweigten Alkylrest mit 6 bis 12, vorzugsweise 8 bis 12, und besonders bevorzugt 11, Kohlenstoffatomen und n im Durchschnitt für eine Zahl von 2 bis 12, vorzugsweise 5 bis 11, besonders bevorzugt 6 bis 9, und insbesondere bevorzugt 8, steht,
b) ein oder mehrere ethoxylierte Triglyceride deren Acylreste sich von linearen oder verzweigten gesättigten Fettsäuren mit 6 bis 22 und vorzugsweise 12 bis 18, Kohlenstoffatomen ableiten, und
c) Wasser,
**dadurch gekennzeichnet, dass** der Lösungsvermittler bei Raumtemperatur flüssig und klar ist und keine Anlagerungsprodukte von Ethylenoxid und gleichzeitig Propylenoxid an Fettalkohole enthält.

2. Lösungsvermittler nach Anspruch 1, **dadurch gekennzeichnet, dass** er mehrere ethoxylierte Fettalkohole gemäß der Formel (I) enthält und das Gewichtsverhältnis von ethoxylierten Fettalkoholen der Formel (I) mit linearen Resten R¹ zu ethoxylierten Fettalkoholen der Formel (I) mit verzweigten Resten R¹ von 40 : 60 bis 60 : 40, vorzugsweise von 45 : 55 bis 55 : 45, und besonders bevorzugt 50 : 50, beträgt.

3. Lösungsvermittler nach Anspruch 1 oder 2, **dadurch gekennzeichnet, dass** R¹ ein linearer oder verzweigter Alkylrest mit 11 Kohlenstoffatomen ist.

4. Lösungsvermittler nach einem oder mehreren der Ansprüche 1 bis 3, **dadurch gekennzeichnet, dass** n im Durchschnitt für eine Zahl von 6 bis 9 und vorzugsweise 8 steht.

5. Lösungsvermittler nach einem oder mehreren der Ansprüche 1 bis 4, **dadurch gekennzeichnet, dass** die ein oder mehreren ethoxylierten Triglyceride der Komponente b) ausgewählt sind aus Anlagerungsprodukten von durchschnittlich 20 bis 100 Mol, vorzugsweise 30 bis 80 Mol, besonders bevorzugt 35 bis 65 Mol, und insbesondere bevorzugt 40 Mol Ethylenoxid an Triglyceride.

6. Lösungsvermittler nach einem oder mehreren der Ansprüche 1 bis 5, **dadurch gekennzeichnet, dass** die ein oder mehreren ethoxylierten Triglyceride der Komponente b) ausgewählt sind aus Anlagerungsprodukten von durchschnittlich 20 bis 100 Mol, vorzugsweise 30 bis 80 Mol, besonders bevorzugt 35 bis 65 Mol, und insbesondere bevorzugt 40 Mol Ethylenoxid an hydriertes Castoröl.

7. Lösungsvermittler nach einem oder mehreren der Ansprüche 1 bis 6, **dadurch gekennzeichnet, dass** er die ethoxylierten Fettalkohole der Komponente a) in einer Menge von 20 bis 70 Gew.-%, vorzugsweise von 30 bis 65 Gew.-%, und besonders bevorzugt von 33 bis 60 Gew.-%, die ethoxylierten Triglyceride der Komponente b) in einer Menge von 20 bis 60 Gew.-%, vorzugsweise von 25 bis 55 Gew.-%, und besonders bevorzugt von 26 bis 51 Gew.-% und Wasser in einer Menge von 1 bis 20 Gew.-%, vorzugsweise von 5 bis 18 Gew.-%, und besonders bevorzugt von 9 bis 16 Gew.-% enthält.

8. Lösungsvermittler nach einem oder mehreren der Ansprüche 1 bis 7, **dadurch gekennzeichnet, dass** er frei ist von Propylenoxid-haltigen Komponenten.

9. Lösungsvermittler nach einem oder mehreren der Ansprüche 1 bis 8, **dadurch gekennzeichnet, dass** er aus den ethoxylierten Fettalkoholen der Komponente a), den ethoxylierten Triglyceriden der Komponente b) und Wasser besteht.

10. Verwendung eines Lösungsvermittlers nach einem oder mehreren der Ansprüche 1 bis 9 zur Herstellung von kosmetischen, dermatologischen oder pharmazeutischen Formulierungen.

11. Kosmetische, dermatologische oder pharmazeutische Formulierung hergestellt unter Verwendung eines Lösungsvermittlers nach einem oder mehreren der Ansprüche 1 bis 9, **dadurch gekennzeichnet, dass** sie
a1) C₁₁-Alkyl-O-(CH₂CH₂O)₈-H,
b1) ethoxyliertes hydriertes Castoröl mit 40 Ethylenoxid-Einheiten, und
c) Wasser enthält und
d) entweder keine Propylenoxid-haltigen Substanzen enthält oder in dem Fall, dass sie Propylenoxid-haltige Substanzen enthält, bezogen auf die fertige Formulierung, 1,0 Gew.-% oder weniger, vorzugsweise 0,05 Gew.-% oder weniger und besonders bevorzugt 0,01 Gew.-% oder weniger, an Propylenoxid-haltigen Substanzen enthält.

12. Kosmetische, dermatologische oder pharmazeutische Formulierung, **dadurch gekennzeichnet, dass** sie
a1) C₁₁-Alkyl-O-(CH₂CH₂O)₈-H,
b1) ethoxyliertes hydriertes Castoröl mit 40 Ethylenoxid-Einheiten, und
c) Wasser enthält und
d) entweder keine Propylenoxid-haltigen Substanzen enthält oder in dem Fall, dass sie Propylenoxid-haltige Substanzen enthält, bezogen auf die fertige Formulierung, 1,0 Gew.-% oder weniger, vorzugsweise 0,05 Gew.-% oder weniger und besonders bevorzugt 0,01 Gew.-% oder weniger, an Propylenoxid-haltigen Substanzen enthält.

13. Formulierung nach Anspruch 11 oder 12, **dadurch gekennzeichnet, dass** sie, bezogen auf die fertige Formulierung, den ethoxylierten Alkohol der Komponente a1) von 0,05 bis 6,0 Gew.-% und das ethoxylierte hydrierte Castoröl mit 40 Ethylenoxid-Einheiten der Komponente b1) von 0,02 bis 5,0 Gew.-% enthält.

14. Formulierung nach einem oder mehreren der Ansprüche 11 bis 13, **dadurch gekennzeichnet, dass** sie keine Propylenoxid-haltigen Substanzen enthält.

15. Formulierung nach einem oder mehreren der Ansprüche 11 bis 14, **dadurch gekennzeichnet, dass** sie, bezogen auf die fertige Formulierung, eine Menge an Wasser von 30,0 Gew.-% oder mehr, bevorzugt von 40,0 bis 95,0 Gew.-%, besonders bevorzugt von 50,0 bis 95,0 Gew.-% und insbesondere bevorzugt von 60,0 bis 95,0 Gew.-%, enthält.

16. Formulierung nach einem oder mehreren der Ansprüche 11 bis 15, **dadurch gekennzeichnet, dass** sie in Wasser schwerlösliche Komponenten, insbesondere ausgewählt aus Parfümölen, Vitaminen und UV-Lichtschutzfiltern, enthält.

17. Formulierung nach Anspruch 16, **dadurch gekennzeichnet, dass** sie, bezogen auf die fertige Formulierung, 0,01 bis 30,0 Gew.-%, vorzugsweise 0,01 bis 20,0 Gew.-%, besonders bevorzugt 0,05 bis 10,0 Gew.-% und insbesondere bevorzugt 0,5 bis 5,0 Gew.-%, an in Wasser schwerlöslichen Komponenten, insbesondere an in Wasser schwerlöslichen Komponenten ausgewählt aus Parfümölen, Vitaminen und UV-Lichtschutzfiltern, enthält.

18. Formulierung nach einem oder mehreren der Ansprüche 11 bis 17, **dadurch gekennzeichnet, dass** sie ein klares Aussehen aufweist. '
